# EUROPEAN PATENT SPECIFICATION

(11) **EP 3 446 745 B1**
(45) Date of publication and mention of the grant of the patent: **19.02.2020**
(21) Application number: 18188648.2
(22) Date of filing: 13.08.2018
(51) Int. Cl.: A61N 1/04, A61N 1/32, A61N 1/36, A61H 23/02, A61B 5/00

(54) **BEAUTY APPARATUS**
SCHÖNHEITSVORRICHTUNG
APPAREIL DE SOINS DE BEAUTÉ

(30) Priority: 24.08.2017 JP 2017161295
(43) Date of publication of application: 27.02.2019
(73) Proprietor: Panasonic Intellectual Property Management Co., Ltd., Osaka-shi, Osaka 540-6207 (JP)
(72) Inventor: MATSUSAKA, Takeshi, Osaka-shi, Osaka 540-6207 (JP); WATANABE, Shunichi, Osaka-shi, Osaka 540-6207 (JP); IBUKI, Yasuo, Osaka-shi, Osaka 540-6207 (JP); ONOGI, Yoko, Osaka-shi, Osaka 540-6207 (JP); TAKECHI, Mitsuru, Osaka-shi, Osaka 540-6207 (JP)
(74) Representative: Grünecker Patent- und Rechtsanwälte PartG mbB

(56) References cited:
- WO-A1-2004/110206
- WO-A1-2014/024335
- WO-A1-2014/147855
- WO-A2-2004/023235
- WO-A2-2012/029065
- WO-A2-2012/100258
- WO-A2-2014/097288
- US-B1- 6 176 840

## Description

### BACKGROUND

### 1. Technical Field

The present disclosure relates to a beauty apparatus used for a skin. The present invention is set out in the appended claims. The embodiments, aspects or examples according to the present description that do not fall within the scope of said claims are provided for illustrative purposes only and do not form part of the present invention.

### 2. Description of the Related Art

Heretofore, a beauty apparatus that can beautify a skin, for example, by applying an ultrasonic wave to the skin has been known (Refer to, for example, as disclosed in Japanese Patent No. 4416028).

In recent years, there is a demand on a beauty apparatus which achieves a further improved treatment efficiency.

Document WO2014097288 discloses an apparatus comprising an array of individually controlled skin treatment units to be applied to a skin segment with each of the units comprising: a housing defining a cavity that fluidly communicates with a source of vacuum pressure, having an inner side terminated by a rim facilitating sealing of the cavity when the unit is applied to the skin, dimensions of the cavity being sufficient to accommodate a volume of a skin segment drawn into the cavity by the source of vacuum pressure to create a skin protrusion.

Document WO2004023235 disclosed a portable beauty care apparatus in which at least two of a low frequency therapy device having two electrodes, an ultrasonic therapy device having vibrator probes, and a 4-electrode type body impedance measuring device are integrated. An integrated electrode structure for integrating the functions of the devices to efficiently and conveniently use the functions is also provided. A measured body impedance is used to calculate a degree of body fat or stress. A structure for incorporating the devices in a mobile communication terminal is also provided.

Document WO2014147855 teaches a cosmetic tool that is capable of applying heat stimulus or cooling stimulus to the skin surface. This cosmetic tool is provided with a heating head on a body case. The heating head includes a tubular head case fixed to the body case, a heat transfer body that covers an outside opening of the head case, and a heat generating structure provided inside the head case. The cylindrical cross-section area of the head case is set so as to decrease from the outside opening toward an attachment base part relative the body case whereby heat from the heat generating structure is prevented from moving toward the body case.

Document WO2012029065 discloses a self-operated device for treating a skin of a patient, comprising: a substrate having a first side and a second side, a plurality of RF electrodes arranged in said substrate, the RF electrodes are configured to emit RF radiation from said first side to the surface of said skin, at least one RF generator configured to generate pulses of current to said RF electrodes and a control unit connected to said at least one RF generator, the control unit is adapted to control the operation of said RF electrodes. The control unit is adapted to control the operation of said RF electrodes according to a predetermined treatment protocol, such that the same activates or deactivates at least one of said RF electrodes at any predetermined time interval according to a predetermined pattern so as to achieve a particular therapeutic result.

### SUMMARY

An object of the present disclosure is to provide a beauty apparatus that can further improve the beauty treatment efficiency.

A beauty apparatus according to an aspect of the present disclosure includes a first stimulation provider that provides a skin with a first stimulation, a second stimulation provider that provides the skin with a second stimulation induced by an electromagnetic change in an radio frequency (RF), and different in type from a first stimulation, a detector that detects a contact of the skin with the first stimulation provider and the second stimulation provider, and a controller that controls the first stimulation provider and the second stimulation provider based on a detection result of the detector.

The present disclosure can provide a beauty apparatus that achieves a further improved treatment efficiency.

### BRIEF DESCRIPTION OF THE DRAWINGS

FIG. 1 is a plan view illustrating a schematic configuration of a beauty apparatus according to an exemplary embodiment;
FIG. 2 is a sectional view illustrating a schematic configuration of a beauty apparatus according to the exemplary embodiment;
FIG. 3 is a plan view illustrating a schematic configuration of a head according to the exemplary embodiment when viewed from the front of a probe head;
FIG. 4 is a sectional view illustrating the schematic configuration of the head according to the exemplary embodiment;
FIG.5 is a perspective view illustrating a schematic configuration of a second holder and a second stimulation provider according to the exemplary embodiment;
FIG. 6 is a perspective view illustrating a state where a part of the second holder according to the exemplary embodiment is detached;
FIG. 7 is a perspective view illustrating a schematic configuration of a terminal according to the exemplary embodiment;
FIG. 8 is a perspective view illustrating a schematic configuration of conductive plate according to the exemplary embodiment;
FIG. 9 is a block diagram illustrating the control configuration of beauty apparatus 10 according to the exemplary embodiment; and
FIG. 10 is a sectional view illustrating a state where a protrusion of the probe head according to the exemplary embodiment is pressed against a skin.

### DETAILED DESCRIPTION

Hereinafter, a beauty apparatus according to an exemplary embodiment of the present disclosure will be described in detail with reference to the drawings. The exemplary embodiment described below is a preferred specific example of the present disclosure. Accordingly, numerical values, shapes, materials, configuration elements, or arrangements and connecting forms of the configuration elements, which are illustrated in the following exemplary embodiment, are merely examples, and do not limit the present disclosure. Therefore, in the configuration elements according to the following exemplary embodiment, the configuration elements which are not described in an independent claim representing a most significant concept of the present disclosure will be described as optional configuration elements.

Each drawing is a schematically illustrated, and is not necessarily strictly illustrated. In the respective drawings, identical components are denoted by identical reference symbols.

### [Beauty apparatus]

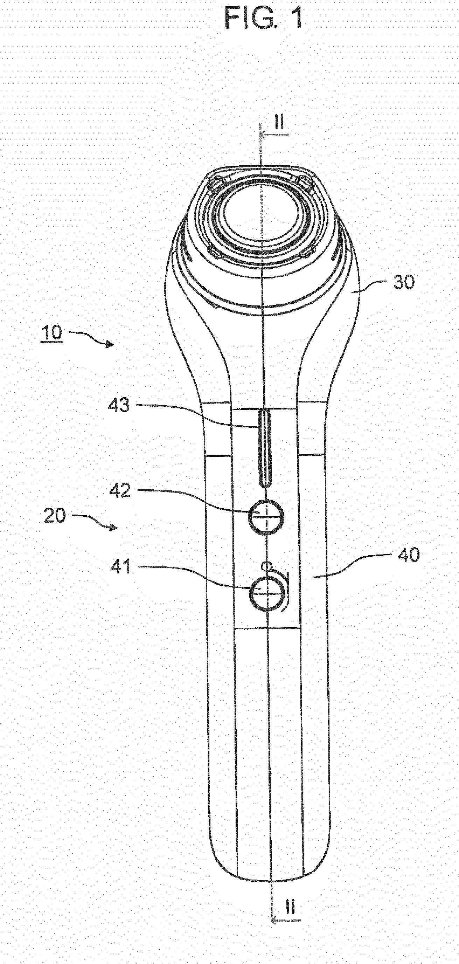

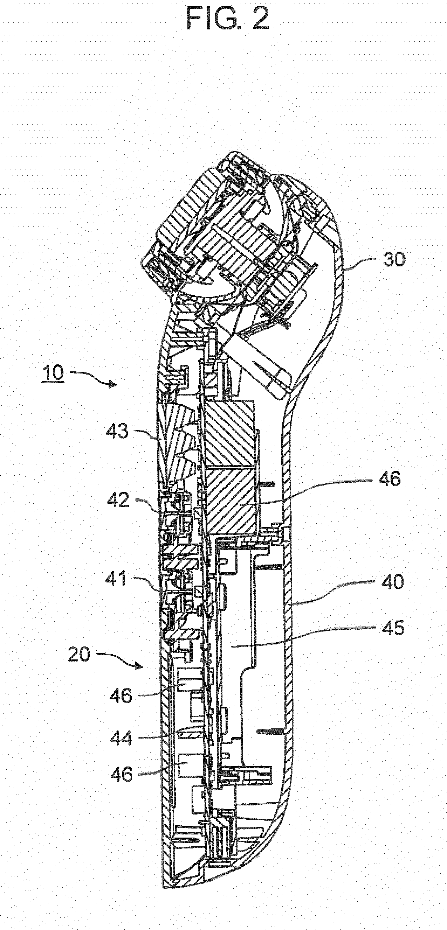

First, beauty apparatus 10 according to an exemplary embodiment will be described. FIG. 1 is a plan view illustrating a schematic configuration of beauty apparatus 10 according to the exemplary embodiment. FIG. 2 is a sectional view illustrating the schematic configuration of beauty apparatus 10 according to the exemplary embodiment. Specifically, FIG. 2 is a sectional view illustrating a section including section line II-II in FIG. 1.

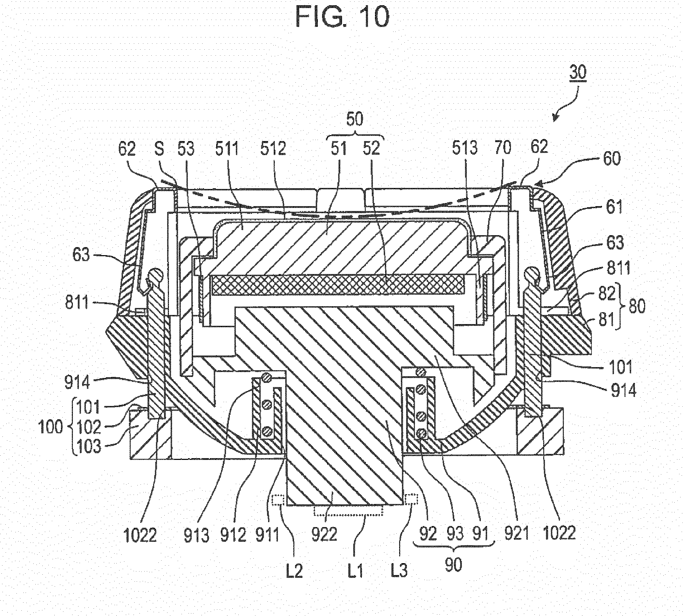

As illustrated in FIGS. 1 and 2, beauty apparatus 10 includes housing 20 extending in one direction, which is an external body. One end portion of housing 20 serves as head 30 that provides skin S with a stimulation (see FIG. 10). A part other than head 30 in housing 20 is gripper 40 gripped by a user, and is formed narrower than head 30. Gripper 40 has power button 41 for switching on (ON) and off (OFF) power, selection button 42 for selecting an operation mode of beauty apparatus 10, and lighting portion 43 that is turned on when the power is switched on and is turned off when the power is switched off.

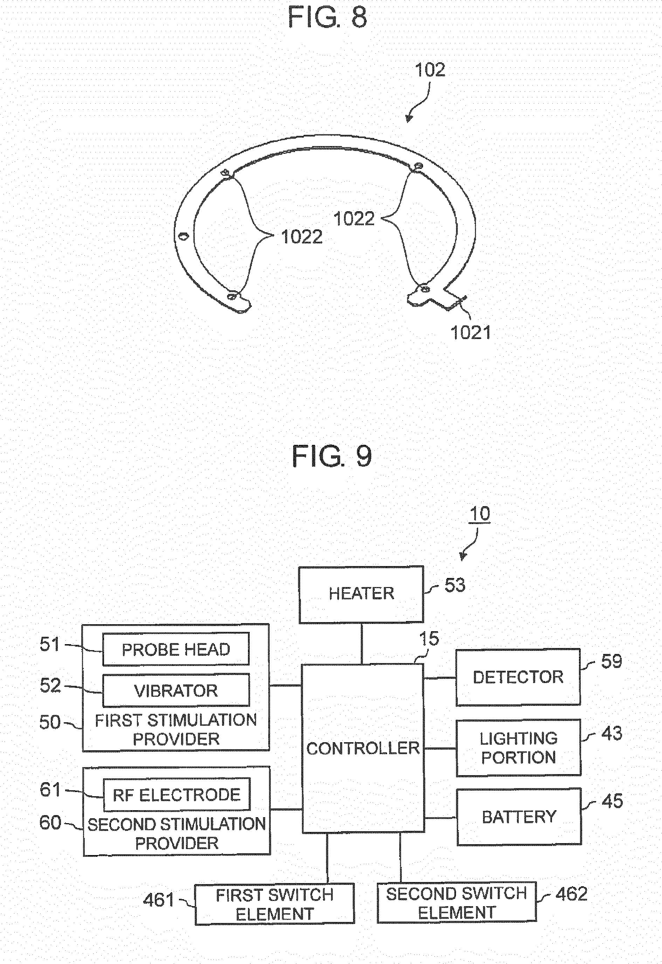

As illustrated in FIG. 2, circuit board 44 and battery 45 are accommodated inside gripper 40. A plurality of circuit components 46 for driving drive units of head 30 are mounted on circuit board 44. The plurality of circuit components 46 include a microcontroller (hereinafter abbreviated as referred to "micon", and the micon is controller 15 (see FIG. 9) for controlling the drive units of beauty apparatus 10.

The plurality of circuit components 46 further include first and second switch elements 461 and 462 (see FIG. 9). First switch element 461 is used for turning on and off power. Pressing down power button 41 of first switch element 461 outputs, to controller 15, a control signal for switching on and off power supply from battery 45. Second switch element 462 is used for selecting the operation mode. Pressing down selection button 42 of second switch element 462 outputs, to controller 15, a control signal corresponding to each operation mode.

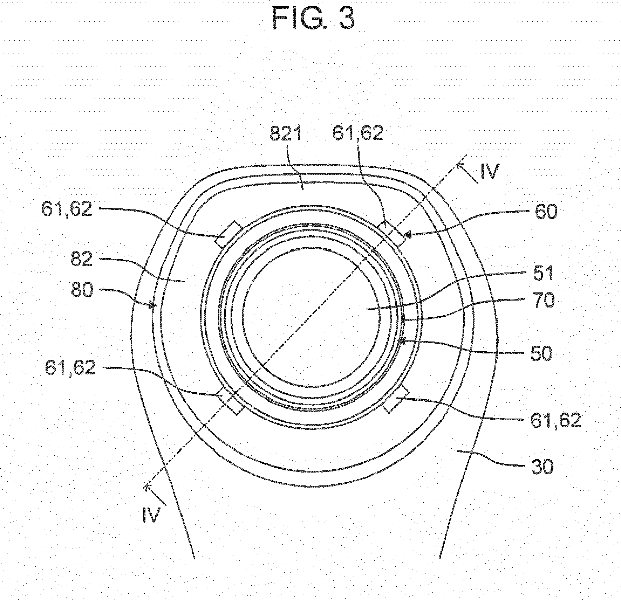

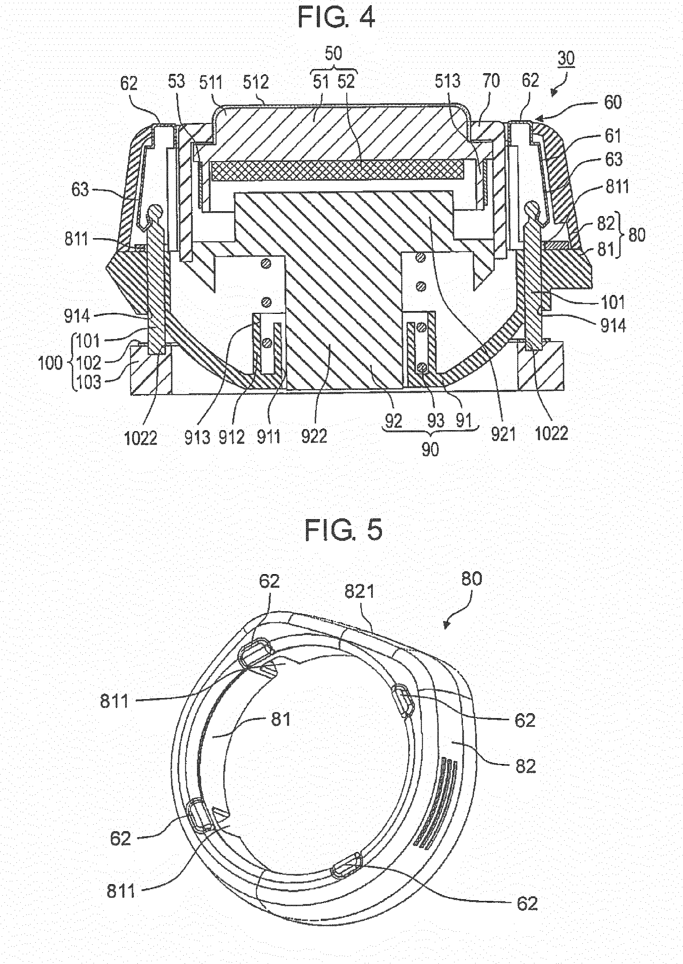

FIG. 3 is a plan view illustrating a schematic configuration of head 30 according to the exemplary embodiment when viewed from the front of a probe head. FIG. 4 is a sectional view illustrating the schematic configuration of head 30 according to the exemplary embodiment. Specifically, FIG. 4 is a sectional view illustrating a section including section line IV-IV in FIG. 3.

As illustrated in FIGS. 3 and 4, head 30 includes first stimulation provider 50, second stimulation provider 60, first holder 70, second holder 80, retractable mechanism 90, and terminal 100. First holder 70 and second holder 80 hold first stimulation provider 50 and second stimulation provider 60, respectively, such that second stimulation provider 60 is disposed close to first stimulation provider 50. In this manner, first and second stimulation providers 50 and 60 can simultaneously come into contact with skin S of the user.

First stimulation provider 50 provides skin S with a first stimulation. The first stimulation includes an ultrasonic wave vibration stimulation, an electrical stimulation (for example, electrical muscle stimulation: EMS), a thermal stimulation, and a light stimulation. In the present exemplary embodiment, the ultrasonic wave vibration stimulation will be described as an example of the first stimulation.

First stimulation provider 50 includes probe head 51 and vibrator 52 that vibrates probe head 51. Probe head 51 is a metal body that comes into contact with skin S and transmits vibration generated by vibrator 52 to skin S. In probe head 51, a portion exposed from head 30 protrudes in a disc shape to form protrusion 511. Oxide film 512 is formed on a surface of protrusion 511. For example, in the present exemplary embodiment, after probe head 51 is formed of aluminum, protrusion 511 is subjected to anodization, whereby oxide film 512 formed of aluminum oxide is generated. A surface of probe head 51 may entirely serve as oxide film 512.

Circularly erected peripheral wall 513 is formed on a side opposite to protrusion 511 in probe head 51. Sheet-like heater 53 is attached to an entire outer peripheral surface of peripheral wall 513. Heater 53 heats skin S via probe head 51 by heating probe head 51. Heater 53 is electrically connected to controller 15, and temperature of heater 53 is adjusted under the control of the controller 15.

Vibrator 52 vibrates probe head 51 by vibrating at a frequency bandwidth of the ultrasound wave, and applies the ultrasound wave vibration stimulation to skin S in contact with probe head 51. Specifically, vibrator 52 is a piezoelectric element, and is fixed to probe head 51 inside a region surrounded by peripheral wall 513. In vibrator 52, the vibration is controlled under the control of the controller 15.

First holder 70 is a resin member that holds first stimulation provider 50. Specifically, first holder 70 is formed in a substantially tubular shape, and is attached to an outer peripheral edge of protruding portion 511 so that protruding portion 511 of probe head 51 protrudes outward from one end of housing 20. First holder 70 is connected to retractable mechanism 90.

Retractable mechanism 90 is a mechanism for retracting first stimulation provider 50 to second stimulation provider 60 by retracting first holder 70 to head 30. Specifically, retractable mechanism 90 includes base 91, connector 92, and elastic member 93.

Base 91 is a resin member configuring a part of the contour of head 30, and is formed in a substantial cup shape. Connector 92 is configured to move relatively to base 91. Specifically, through-hole 911 is formed at the center of base 91, and tubular portion 912 concentric with through-hole 911 is formed around through-hole 911. Tubular portion 912 has accommodation groove 913 that accommodates elastic member 93. Accommodation groove 913 is formed concentrically with through hole 911. A plurality of attachment holes 914 are formed on an outer peripheral portion of base 91. A plurality of electrode pins 101 included in terminal 100 are individually attached to the plurality of attachment holes 914.

Connector 92 is a resin member connected to first holder 70. Connector 92 includes: fitting portion 921 fitted to first holder 70, and shaft 922 protruding from fitting portion 921 and inserted into through hole 911 of base 91.

Elastic member 93 applies an urging force, which goes outward of head 30, to connector 92 when elastically deformed. Specifically, elastic member 93 is a coil spring. One end portion of the elastic member 93 is fixed into accommodation groove 913, and the other end portion is fixed to fitting portion 921 of connector 92. That is, elastic member 93 is configured to extend and contract by being elastically deformed and elastically restored in a state where elastic member 93 is accommodated in accommodation groove 913. Elastic member 93 may be a spring other than the coil spring, or may be other elastic bodies such as rubber. A retracting operation performed by retractable mechanism 90 will be described later.

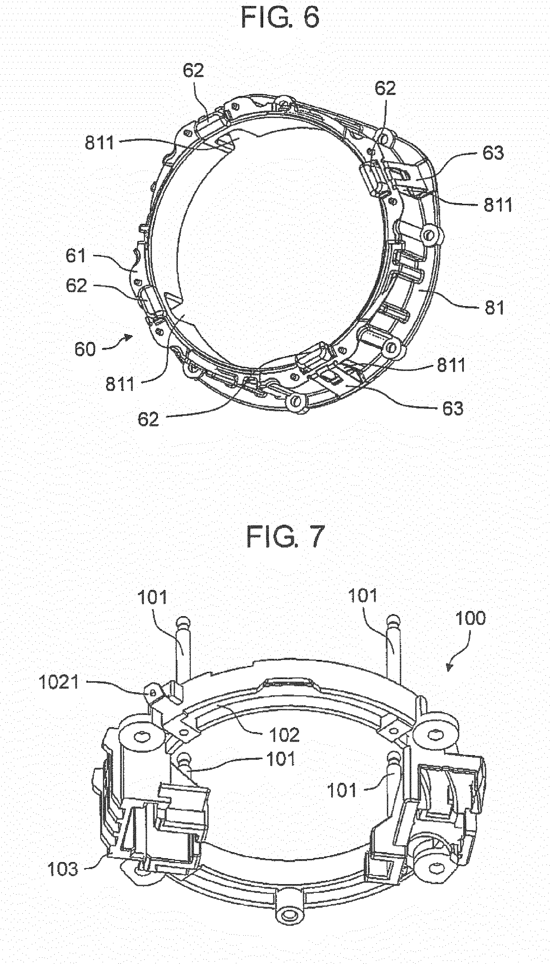

FIG. 5 is a perspective view illustrating a schematic configuration of second holder 80 and second stimulation provider 60 according to the exemplary embodiment. FIG. 6 is a perspective view illustrating a state where a part of the second holder 80 according to the exemplary embodiment is detached.

As illustrated in FIGS. 4 to 6, second stimulation provider 60 is integrally assembled with second holder 80, and second holder 80 is detachable from first holder 70 in a state where second stimulation provider 60 is integrally assembled with second holder 80. Specifically, second holder 80 is configured to be detachable from base 91 for first holder 70.

Second stimulation provider 60 provides skin S with a second stimulation induced by an electromagnetic change in a radio frequency (hereinafter, referred to as an "RF"), and different in type from the first stimulation. Being different in type means that physical phenomena inducing the stimulations are different. Those that bring an electromagnetic change in the RF are an RF current, an RF voltage, an RF electromagnetic wave, and so on. Examples of the second stimulation include a stimulation for heating a deep portion of skin S by generating an electric field or a magnetic field in a range from a surface of skin S to an inside of skin S using the electromagnetic change in the RF. In the present exemplary embodiment, as an example of the second stimulation, a stimulation induced by the electric field generated by the electromagnetic change in the RF will be described.

Specifically, as illustrated in FIG. 6, second stimulation provider 60 includes RF electrode 61 formed in an annular shape. RF electrode 61 is formed of conductive metal. For example, in the present exemplary embodiment, RF electrode 61 is formed of titanium. RF electrode 61 has a plurality of projections 62 protruding outwards from second holder 80. The plurality of projections 62 are arranged at an equal interval in a circumferential direction of RF electrode 61. In the present exemplary embodiment, as illustrated in FIG. 3, four projections are provided, and a portion of each projection 62 exposed outwards from second holder 80 is rectangular in plan view. However, any number and shape of projections 62 may be provided.

As illustrated in FIGS. 4 to 6, locking pieces 63 extending in a direction opposite to a projecting direction of projections 62 are formed on portions of RF electrode 61, the portions facing projections 62. Therefore, RF electrode 61 includes locking pieces 63 having the same number as the number of the plurality of projections 62. Locking pieces 63 are formed in a plate spring shape, and tip ends thereof are bent. Electrode pins 101 of terminal 100 are locked to the tip ends of locking pieces 63 (see FIG. 4).

In a case where second stimulation provider 60 provides skin S with the stimulation induced by the electric field generated by the RF, the other RF electrode different in polarity from RF electrode 61 is required. In the present exemplary embodiment, probe head 51 of first stimulation provider 50 also serves as the other RF electrode. In this manner, as a whole, head 30 includes a pair of RF electrodes (RF electrode 61 and probe head 51) having different polarities.

Here, an electromagnetic wave in a frequency band of 10 kHz or more to 100 GHz or less can be used as that brings the electromagnetic change in the RF. From a viewpoint of a beautifying effect, it is more desirable to use an electromagnetic wave or a current in a frequency band of 0.3 MHz or more to 50 MHz or less. The present exemplary embodiment employs a current at approximately 1 MHz, which is suitable for aluminum-made probe head 51 provided with oxide film 512.

Second holder 80 is a member holding second stimulation provider 60 and freely detachable from first holder 70. Specifically, second holder 80 has an annular outline as a whole (see FIG. 5). A first stimulation provider 50 is disposed in an opening of second holder 80 (see FIG. 4). Second holder 80 includes frame 81 and cover 82. Frame 81 is an annularly formed resin member, and holds RF electrode 61 of second stimulation provider 60. Frame 81 has guide route 811 that guides electrode pin 101 of terminal 100 to each locking piece 63 of RF electrode 61. Cover 82 is a resin member formed annularly. Cover 82 is attached to frame 81, thereby covering the other portion of RF electrode 61 in a state where projections 62 of RF electrode 61 are individually exposed. A portion of an edge portion of cover 82 is formed in a flat shape. The portion is referred to as flat portion 821. Flat portion 821 is located in a tip end portion of housing 20, and is disposed along a direction orthogonal to a longitudinal direction of housing 20. For example, in a case where an opening portion of cover 82 is circular, flat portion 821 is formed in a flat shape substantially parallel to a tangent line of the opening portion. In this manner, flat portion 821 in cover 82 is disposed closer to first stimulation provider 50 and second stimulation provider 60 than a portion other than flat portion 821 in cover 82 is. Specifically, as illustrated in FIG. 3, entire flat portion 821 is adjacent to probe head 51 of first stimulation provider 50. Projections 62 of second stimulation provider 60 face both respective ends of flat portion 821.

FIG. 7 is a perspective view illustrating a schematic configuration of terminal 100 according to the exemplary embodiment.

Terminal 100 is a region for supplying power to second stimulation provider 60 by being electrically connected to second stimulation provider 60 (see FIG. 4). Specifically, as illustrated in FIG. 7, terminal 100 includes the plurality of electrode pins 101, conductive plate 102, and bracket 103.

Each of electrode pins 101 is formed in a columnar shape, and has a tip end formed in a substantially spherical shape. The tip ends of electrode pins 101 protrude from attachment holes 914 of base 91. Locking piece 63 of RF electrode 61 is locked to the tip end portion of electrode pin 101, thereby bringing second holder 80 into a state where second holder 80 is attached to first holder 70 (see FIG. 4). In this state, RF electrode 61 and electrode pins 101 are electrically connected to each other.

Meanwhile, if second holder 80 is pulled in a direction away from first holder 70, then the tip ends of electrode pins 101 and locking pieces 63 are unlocked from each other, and second holder 80 is detached from first holder 70 (see FIG. 5). In this state, RF electrode 61 and electrode pins 101 are not electrically connected to each other.

FIG. 8 is a perspective view illustrating a schematic configuration of conductive plate 102 according to the exemplary embodiment. FIG. 8 is a perspective view of conductive plate 102 when viewed in a direction different from that in FIG. 7. As illustrated in FIG. 8, conductive plate 102 is a conductive metal plate formed in a substantially C shape. Flat plate-like tongue portion 1021 protruding outward is formed in one end of conductive plate 102. Tongue portion 1021 is electrically connected to power supplying circuit component 46 via a wire (not illustrated). A C-shaped portion of conductive plate 102 has a plurality of connection holes 1022 to which the plurality of electrode pins 101 are individually connected. When electrode pins 101 are connected to connection holes 1022, conductive plate 102 and electrode pins 101 are electrically connected to each other. As mentioned above, each of the plurality of electrode pins 101 is electrically connected to RF electrode 61 of second stimulation provider 60. Accordingly, conductive plate 102 and RF electrode 61 are electrically connected to each other via electrode pins 101. That is, terminal 100 and second stimulation provider 60 are electrically connected to each other at a plurality of locations. Accordingly, even if one of electrode pins 101 is broken and is not in an electrically connected state, the electrically connected state is maintained by other remaining electrode pins 101.

As illustrated in FIG. 7, bracket 103 is a member that holds the plurality of electrode pins 101 and conductive plate 102. Bracket 103 is formed annularly, and a part of retractable mechanism 90 is disposed inside an opening portion of bracket 103 (see FIG. 4). Bracket 103 is fixed into housing 20.

### [Control configuration]

Next, a control configuration of beauty apparatus 10 will be described. FIG. 9 is a block diagram illustrating the control configuration of beauty apparatus 10 according to the exemplary embodiment.

Controller 15 of beauty apparatus 10 in FIG. 9 is, for example, a micon including a timer. To controller 15, there are electrically connected first stimulation provider 50, second stimulation provider 60, first switch element 461, second switch element 462, lighting portion 43, battery 45, heater 53, and detector 59. Based on the control signals output from first and second switch elements 461 and 462 and on a detection result of detector 59, controller 15 controls first stimulation provider 50, second stimulation provider 60, lighting portion 43, battery 45, and heater 53. Specifically, upon receiving the control signal from first switch element 461 in such a manner that power button 41 is operated when a power source is turned off, controller 15 supplies the respective portions with the power from battery 45 that is the power source. Meanwhile, upon receiving the control signal from first switch element 461 in such a manner that power button 41 is operated when the power source is turned on, controller 15 stops supplying the respective portions with power from battery 45. Controller 15 turns on lighting portion 43 when the power source is turned on, and turns off lighting portion 43 when the power source is turned off.

Upon receiving the control signal corresponding to each of the operation modes from second switch element 462 in such a manner that selection button 42 is operated when the power source is turned on, controller 15 controls first stimulation provider 50, second stimulation provider 60, and heater 53 in response to the operation mode concerned. In any of the operation modes, controller 15 controls first stimulation provider 50, second stimulation provider 60, and heater 53, based on the detection result of detector 59. The operation modes include a mode in which at least one of first stimulation provider 50 and second stimulation provider 60 continuously operates, and a mode in which at least one of first stimulation provider 50 and second stimulation provider 60 intermittently operates.

Detector 59 is a sensor that detects the contact of skin S with first stimulation provider 50 and second stimulation provider 60. Detector 59 can employ any of a mechanical system, an optical system, and an electrical system.

First, a case where detector 59 is configured of the mechanical system will be described.

FIG. 10 is a sectional view illustrating a state where protrusion 511 of probe head 51 according to the exemplary embodiment is pressed against a skin S. Specifically, FIG. 10 corresponds to FIG. 4. In FIG. 10, an outline of skin S is illustrated by a two-dot chain line.

If protruding portion 511 of probe head 51 is pressed against skin S in a state illustrated in FIG. 4, probe head 51 descends from head 30, and elastic member 93 is contracted as illustrated in FIG. 10. When skin S is brought into contact with both of probe head 51 and projections 62 of RF electrode 61, probe head 51 stops descending. The switch element as detector 59 is disposed at a spot against which any one of first holder 70, first stimulation provider 50, and retractable mechanism 90 abuts when probe head 51 stops descending. In the presence of abutting against the detector 59, detector 59 outputs a detection signal to controller 15. Meanwhile, in the absence of abutting against detector 59, detector 59 does not detect a detection signal. That is, a state where detector 59 outputs the detection signal indicates that skin S is in contact with both of probe head 51 and projections 62 of RF electrode 61.

For example, detector 59 which is a mechanical type is disposed on a spot (see a broken line L1 in FIG. 10) against which a tip end surface of shaft 922 of connector 92 abuts when skin S is brought into contact with both of probe head 51 and projections 62 of RF electrode 61.

Next, a case where detector 59 is configured of the optical system will be described.

Optical detector 59 includes a light emitter and a light receiver, and outputs different signals depending on whether a current state is switched between a light receiving state where the light receiver receives light from the light emitter and a non-light receiving state where the light receiver does not receive the light from the light emitter. In the present exemplary embodiment, such a signal output in the non-light receiving state is defined as the detection signal. The light emitter and the light receiver are disposed at spots where the light from the light emitter to the light receiver is not blocked in the state illustrated in FIG. 4 and where the light from the light emitter to the light receiver is blocked in the state illustrated in FIG. 10. In this manner, when skin S is brought into contact with both of probe head 51 and projections 62 of RF electrode 61 and probe head 51 stops descending, then the light from the light emitter is blocked to bring the non-light receiving state, and the detection signal is output from detector 59 to controller 15.

For example, in optical detector 59, the pair of the light emitter and the light receiver are disposed at spots (see broken lines L2 and L3 in FIG. 10) where shaft 922 of connector 92 enters between the light emitter and the light receiver and blocks light when skin S is brought into contact with both of probe head 51 and projections 62 of RF electrode 61.

Next, a case where detector 59 is configured of the electrical system will be described.

Electrical detector 59 outputs the detection signal to controller 15 in a case where the pair of RF electrodes (probe head 51 and RF electrode 61) are electrically connected to each other via skin S when the RF electrodes are brought into contact with skin S. Specifically, detector 59 measures a current value between the pair of RF electrodes. When the current value exceeds a threshold, detector 59 determines that the pair of RF electrodes is electrically connected to each other via skin S, and outputs the detection signal to controller 15.

Controller 15 controls first stimulation provider 50, second stimulation provider 60, and heater 53, based on the detection result of detector 59. Specifically, when the detection signal output from detector 59 is not present, that is, when detector 59 does not detect the contact of skin S, controller 15 stops the operation of first stimulation provider 50, second stimulation provider 60, and heater 53. At this time, if a state in which detector 59 does not detect the contact of skin S continues for a second predetermined time when the power source is turned on, controller 15 stops supplying the portions with power from battery 45, and turns off the power source.

Meanwhile, when the detection signal output from detector 59 is present, that is, when detector 59 detects the contact of skin S, controller 15 operates first stimulation provider 50, second stimulation provider 60, and heater 53. At this time, if the operation of first stimulation provider 50, second stimulation provider 60, and heater 53 continues for a first predetermined time, controller 15 stops the operation.

When the detection signal output from detector 59 is present, controller 15 blinks lighting portion 43. When the detection signal output from detector 59 is gone, controller 15 lights lighting portion 43. As described above, a lighting condition of lighting portion 43 changes depending on whether the detection signal is present. Accordingly, the user can be notified whether skin S is in contact with both of probe head 51 and projections 62 of RF electrode 61.

### [Operations]

Next, operations of beauty apparatus 10 will be described.

First, the user applies moisture-containing cosmetics or treatment gel to at least one of skin S to be treated and probe head 51 of beauty apparatus 10. Thereafter, if the user operates power button 41, the power source of beauty apparatus 10 is turned on. At this time, controller 15 controls and lights lighting portion 43.

Immediately after power is supplied, as illustrated in FIG. 4, elastic member 93 of retractable mechanism 90 is in a stretched state, and protruding portion 511 of probe head 51 protrudes from head 30.

Next, if the user operates selection button 42, controller 15 prepares for the control in an operation mode desired by the user. Thereafter, the user then presses protrusion 511 of probe head 51 against skin S. When probe head 51 descends from head 30, and skin S is brought into contact with protrusion 511 of probe head 51 and projections 62 of RF electrode 61 as illustrated in FIG. 10, then the detection signal indicating the contact of skin S is output from detector 59 to controller 15.

When the detection signal is input, controller 15 controls first stimulation provider 50, second stimulation provider 60, and heater 53, thereby causing them to operate in the prepared operation mode. In this manner, three stimulations are provided to skin S.

Each of the stimulations will be specifically described below.

The first stimulation is the ultrasonic wave vibration induced by vibrator 52. The ultrasonic wave vibration generated by vibrator 52 is provided to skin S via probe head 51. The ultrasonic wave vibration is provided to skin S, thereby allowing moisture distribution inside skin S to be uniform. Accordingly, tension can be provided to skin S.

The second stimulation is a stimulation based on the electromagnetic field, which is induced by the RF current generated by the pair of RF electrodes (probe head 51 and RF electrode 61). When both of probe head 51 and RF electrode 61 come into contact with skin S in a state of being applied with a voltage in the frequency band where the RF is generated, an electric field is generated at a specific region of skin S, and Joule heat generated by a current is generated in the region. In this way, if the specific region of skin S is heated, skin S can be activated by promoted blood flow and action of gene expression.

The third stimulation is heat induced by heater 53. If heater 53 is heated, probe head 51 in contact with heater 53 is also heated. In this manner, the heat is transferred from probe head 51 to skin S, and skin S is warmed. When skin S is warmed, the current becomes easy to flow in skin S. Accordingly, it becomes easy to generate the electric field, which is induced by the RF, in skin S, and the current becomes easy to flow. Hence, it becomes easier to warm the deep portion of skin S to improve a heating effect.

Note that, upon receiving the detection signal, controller 15 controls and blinks lighting portion 43. The user sees the blink of lighting portion 43 directly or via a mirror, and can thereby get to know that skin S comes into contact with protrusion 511 of probe head 51 and projections 62 of RF electrode 61 and is provided with the respective stimulations.

In a state where probe head 51 is pressed against skin S, the user moves beauty apparatus 10 such that probe head 51 slides on skin S. In this case, if beauty apparatus 10 is moved in a direction where flat portion 821 moves forward, the user can obtain a more lifted feeling on skin S.

Here, if the operation of first stimulation provider 50, second stimulation provider 60, and heater 53 continues for a first predetermined time, controller 15 stops the operation. This prevents the stimulations from being provided to skin S for the first predetermined time or longer. Excessive stimulations can be prevented from being provided to skin S by setting the first predetermined time such that excessive stimulations are not supplied to skin S.

When the contact of skin S with probe head 51 is released in the state illustrated in FIG. 10 by the operation of the user, elastic member 93 is elastically restored to extend as illustrated in FIG. 4, and probe head 51 returns to an original position of itself. In this state, probe head 51 protrudes from head 30, and accordingly, detector 59 does not output the detection signal. When the input of the detection signal to controller 15 from detector 59 is stopped, controller 15 stops the operation of first stimulation provider 50, second stimulation provider 60, and heater 53. When the input of the detection signal to controller 15 is stopped, controller 15 controls and lights lighting portion 43.

For example, in a case where beauty apparatus 10 is moved on skin S, when the lighting state of lighting portion 43 is switched from blinking to lighting, the user recognizes that beauty apparatus 10 is not correctly pressed against skin S. For this reason, the user correctly presses beauty apparatus 10 against skin S and then, moves beauty apparatus 10 again. That is, useless movement of beauty apparatus 10 can be reduced.

Further, if a state in which detector 59 does not output the detection signal, that is, a state in which detector 59 does not detect the contact of skin S continues for a second predetermined time, controller 15 stops supplying the portions with power from battery 45, and turns off power supply. Thereby, wasteful consumption of power can be prevented.

### [Effects]

As described above, beauty apparatus 10 according to the exemplary embodiment includes first stimulation provider 50 that provides skin S with the first stimulation, second stimulation provider 60 that provides the skin with a second stimulation induced by the electric field or the magnetic field of the RF, and different in type from the first stimulation, detector 59 that detects the contact of skin S with first stimulation provider 50 and second stimulation provider 60, and controller 15 that controls first stimulation provider 50 and second stimulation provider 60, based on the detection result of detector 59.

In this manner, controller 15 controls first stimulation provider 50 and second stimulation provider 60 based on the detection result of detector 59. For this reason, controller 15 can properly control first stimulation provider 50 and second stimulation provider 60 depending on whether the contact of skin S with first stimulation provider 50 and second stimulation provider 60 is present, thereby improving the treatment efficiency.

Controller 15 operates first stimulation provider 50 and second stimulation provider 60 when detector 59 detects the contact of skin S, and stops the operation of first stimulation provider 50 and second stimulation provider 60 when detector 59 does not detect the contact of skin S.

In this manner, controller 15 operates first stimulation provider 50 and second stimulation provider 60 when skin S is in contact with first stimulation provider 50 and second stimulation provider 60. Accordingly, stimulations can be properly provided to skin S. Meanwhile, controller 15 stops the operation of first stimulation provider 50 and second stimulation provider 60 when skin S is not in contact with first stimulation provider 50 and second stimulation provider 60, thereby preventing any useless operation in a state where the treatment effect is low. Whereby, the treatment efficiency can be further improved.

If the operation of first stimulation provider 50, second stimulation provider 60, and heater 53 continues for a first predetermined time, controller 15 stops the operation.

In this manner, when the operation of first stimulation provider 50 and second stimulation provider 60 continues for a first predetermined time, the operation is stopped, thereby preventing stimulations from being provided to skin S for the first predetermined time or longer. Accordingly, excessive stimulations can be prevented from being provided to skin S to apply suitable treatment to skin S.

Beauty apparatus 10 includes a notification portion (lighting portion 43), and controller 15 controls the notification portion based on the detection result of detector 59.

Since controller 15 controls lighting portion 43 based on the detection result of detector 59, different notification can be made depending whether the contact of skin S with first stimulation provider 50 and second stimulation provider 60 is present. Through such notification, the user can know whether skin S is properly in contact with first stimulation provider 50 and second stimulation provider 60. This can promote the user to properly use beauty apparatus 10, further improving the treatment effect.

Beauty apparatus 10 further includes a power source (battery 45) for supplying power with first stimulation provider 50 and second stimulation provider 60, and controller 15 turns off the power source if the case where detector 59 does not detect the contact of skin S for a second predetermined time.

In this manner, if the state where detector 59 does not detect the contact of skin S continues for the second predetermined time when the power source is on, controller 15 turns off the power source. Accordingly, for example, even in a case where beauty apparatus 10 is left behind when the power source is on, if the state continues for the second predetermined time, the power source is automatically turned off. Accordingly, wasteful consumption of power can be prevented. Meanwhile, in the state where detector 59 detects the contact of skin S, that is, during treatment, the power-on state continues, thereby preventing the power source from being turned off during treatment.

### [Others]

The beauty apparatus according to the present disclosure has been described above based on the exemplary embodiment. However, the present disclosure is not limited to the above exemplary embodiment.

For example, in the above-described exemplary embodiment, a case has been described as an example where probe head 51 also serving as one of the pair of RF electrodes is formed of aluminum. However, probe head 51 may be formed of another metal. The other metal includes stainless steel and titanium. Even in a case of using the other metal, it is preferable to produce the oxide film on the surface of the metal. In this exemplary embodiment, a case has been described as an example where probe head 51 of first stimulation provider 50 also serves as one of the pair of RF electrodes. However, a dedicated member serving as the RF electrode may be provided. Even in a case of using the dedicated member serving as the RF electrode, it is preferable to produce the oxide film on the surface. The production of the oxide film decreases the conductivity of the RF electrode. However, if the frequency band of the voltage supplied to the pair of RF electrodes is set to 0.3 MHz or more, certain conductivity can be ensured for the RF electrode even if the oxide film is present.

In the above-described exemplary embodiment, a case has been described as an example where the electric field is generated from the surface of skin S to the inner side of skin S by the pair of RF electrodes so that the current generates Joule heat. However, the second stimulation provider is also capable of providing skin S with a thermal action or a blood circulation promoting action by generating the magnetic field in skin S using one RF electrode. That is, the second stimulation in this case is induced by the magnetic field.

In the above-described exemplary embodiment, a case has been described as an example where one of the RF electrodes (probe head 51) of the pair of RF electrodes has oxide film 512 and other RF electrode 61 has no oxide film. However, the oxide film may be disposed in the other RF electrode. In this manner, corrosion resistance of the other RF electrode can be improved.

In the above-described exemplary embodiment, a case has been described as an example where retractable mechanism 90 allows first stimulation provider 50 to retract with respect to second stimulation provider 60. However, the retractable mechanism may allow the second stimulation provider to retract with respect to the first stimulation provider. In this case, it is desirable that the first stimulation provider be fixed to the housing. Even in this case, both of the first stimulation provider and the second stimulation provider can be reliably in close contact with skin S.

Moreover, in the above exemplary embodiment, the case where probe head 51 also serves as the RF electrode is described as an example. However, each of a pair of the RF electrodes may be a member dedicated for the RF electrode. In this case, the second stimulation provider includes the pair of RF electrodes. The pair of RF electrodes can generate the electric field inside skin S, so that the current can generate Joule heat. In this case, probe head 51 is usable as a dedicated member for the ultrasonic wave vibration.

In the above exemplary embodiment, lighting portion 43 for visual notification is described as an example of a notification portion. However, other notification portions are usable as long as the notification units can issue notice to the user. The other notification portions include, for example, a speaker for audio notification and a vibrator for tactile notification. A plurality of notification portions different in type are usable in combination.

In the above exemplary embodiment, controller 15 controls first stimulation provider 50 and second stimulation provider 60, as well as heater 53, based on the detection result of detector 59. However, controller 15 may control only first stimulation provider 50 and second stimulation provider 60, based on the detection result of detector 59.

In the above exemplary embodiment, battery 45 is described as an example of the power source. However, a power source circuit connected to a commercial power source may be used as the power source.

Alternatively, the present disclosure includes a form obtained by adopting various modifications which are conceivable by those skilled in the related art for each exemplary embodiment, or a form realized by optionally combining the configuration elements and functions in each exemplary embodiment within the scope not departing from the gist of the present disclosure.

The present disclosure is applicable to the beauty apparatus used for the skin.
- 10: beauty apparatus
- 15: controller
- 20: housing
- 30: head
- 40: gripper
- 41: power button
- 42: selection button
- 43: lighting portion (notification portion)
- 44: circuit board
- 45: Battery (power source)
- 46: circuit component
- 50: first stimulation provider
- 51: probe head
- 52: vibrator
- 53: heater
- 59: detector
- 60: second stimulation provider
- 61: RF electrode
- 62: projection
- 63: locking piece
- 70: first holder
- 80: second holder
- 81: frame
- 82: cover
- 90: retractable mechanism
- 91: base
- 92: connector
- 93: elastic member
- 100: terminal
- 101: electrode pin
- 102: conductive plate
- 103: bracket
- 461: first switch element
- 462: second switch element
- 511: protruding portion
- 512: oxide film
- 513: peripheral wall
- 811: guide route
- 821: flat portion
- 911: through-hole
- 912: tubular portion
- 913: accommodation groove
- 914: attachment holes
- 921: fitting portion
- 922: shaft
- 1021: tongue portion
- 1022: connection holes
- S: skin

## Claims

1. A beauty apparatus (10) comprising:
a first stimulation provider (50) configured to provide a skin with a first stimulation and comprising a probe head (51);
a second stimulation provider (60) configured to provide the skin with a second stimulation induced by an electromagnetic change in a radio frequency (RF), and different in type from the first stimulation;
a detector (59) configured to detect a contact of the skin with the first stimulation provider (50) and the second stimulation provider (60);
a controller (15) configured to control the first stimulation provider (50) and the second stimulation provider (60), based on a detection result of the detector (59), and a retractable mechanism (90) for retracting the first stimulation provider (50) in relation to the second stimulation provider (60) when the probe head (51) of the first stimulation provider (50) is pressed against the skin, the retractable mechanism (90) being configured to stop the retracting when the detector (59) detects contact of the skin with both the first stimulation provider (50) and the second stimulation provider (60).

2. The beauty apparatus (10) according to claim 1,
wherein the controller (15) operates the first stimulation provider (50) and the second stimulation provider (60) when the detector (59) detects the contact of the skin, and stops the operation of the first stimulation provider (50) and the second stimulation provider (60) when the detector (59) does not detect the contact of the skin.

3. The beauty apparatus (10) according to claim 2, wherein
when the operation of the first stimulation provider (50) and the second stimulation provider (60) continues for a first predetermined time, the controller (15) stops the operation of the first stimulation provider and the second stimulation provider (60).

4. The beauty apparatus (10) according to any one of claims 1 to 3, further comprising a notification portion, wherein the controller (15) controls the notification portion, based on a detection result of the detector (59).

5. The beauty apparatus (10) according to any one of claims 1 to 4, further comprising a power source that supplies power to the first stimulation provider (50) and the second stimulation provider (60), wherein when a state in which the power source is turned on and the detector (59) does not detect the contact of the skin continues for a second predetermined time, the controller (15) turns off the power source.

## Patentansprüche

1. Schönheitsvorrichtung (10), die umfasst:
eine erste Stimulationsbereitstellungseinrichtung (50), die konfiguriert ist, um einer Haut eine erste Stimulation bereitzustellen, und die einen Sondenkopf (51) umfasst;
eine zweite Stimulationsbereitstellungseinrichtung (60), die konfiguriert ist, um der Haut eine zweite Stimulation bereitzustellen, die durch eine elektromagnetische Änderung einer Hochfrequenz (HF) induziert wird und sich in der Art von der ersten Stimulation unterscheidet;
einen Detektor (59), der konfiguriert ist, um einen Kontakt der Haut mit der ersten Stimulationsbereitstellungseinrichtung (50) und der zweiten Stimulationsbereitstellungseinrichtung (60) zu erfassen;
eine Steuerung (15), die konfiguriert ist, um die erste Stimulationsbereitstellungseinrichtung (50) und die zweite Stimulationsbereitstellungseinrichtung (60) auf der Basis eines Erfassungsergebnisses des Detektors (59) zu steuern, und
einen zurückziehbaren Mechanismus (90) zum Zurückziehen der ersten Stimulationsbereitstellungseinrichtung (50) im Verhältnis zur zweiten Stimulationsbereitstellungseinrichtung (60), wenn der Sondenkopf (51) der ersten Stimulationsbereitstellungseinrichtung (50) gegen die Haut gedrückt wird, wobei der zurückziehbare Mechanismus (90) konfiguriert ist, um das Zurückziehen anzuhalten, wenn der Detektor (59) den Kontakt der Haut sowohl mit der ersten Stimulationsbereitstellungseinrichtung (50) als auch mit der zweiten Stimulationsbereitstellungseinrichtung (60) erfasst.

2. Schönheitsvorrichtung (10) nach Anspruch 1,
wobei die Steuerung (15) die erste Stimulationsbereitstellungseinrichtung (50) und die zweite Stimulationsbereitstellungseinrichtung (60) betreibt, wenn der Detektor (59) den Kontakt der Haut erfasst, und den Betrieb der ersten Stimulationsbereitstellungseinrichtung (50) und der zweiten Stimulationsbereitstellungseinrichtung (60) unterbricht, wenn der Detektor (59) den Kontakt der Haut nicht erfasst.

3. Schönheitsvorrichtung (10) nach Anspruch 2, wobei,
wenn der Betrieb der ersten Stimulationsbereitstellungseinrichtung (50) und der zweiten Stimulationsbereitstellungseinrichtung (60) für eine erste vorgegebene Zeit fortgesetzt wird, die Steuerung (15) den Betrieb der ersten Stimulationsbereitstellungseinrichtung und der zweiten Stimulationsbereitstellungseinrichtung (60) anhält.

4. Schönheitsvorrichtung (10) nach einem der Ansprüche 1 bis 3, die des Weiteren ein Meldungsteil umfasst, wobei die Steuerung (15) das Meldungsteil auf der Basis eines Erfassungsergebnisses des Detektors (59) steuert.

5. Schönheitsvorrichtung (10) nach einem der Ansprüche 1 bis 4, die des Weiteren eine Energiequelle umfasst, die der ersten Stimulationsbereitstellungseinrichtung (50) und der zweiten Stimulationsbereitstellungseinrichtung (60) Energie liefert, wobei die Steuerung (15) die Energiequelle abschaltet, wenn ein Zustand, in dem die Energiequelle eingeschaltet ist und der Detektor (59) den Kontakt der Haut nicht erfasst, für eine zweite vorgegebene Zeit fortgesetzt wird.

## Revendications

1. Appareil de soin esthétique (10) comprenant :
un premier dispositif de fourniture de stimulation (50) configuré pour fournir à une peau une première stimulation et comprenant une tête de sonde (51) ;
un second dispositif de fourniture de stimulation (60) configuré pour fournir à la peau une seconde stimulation induite par un changement électromagnétique dans une radiofréquence (RF), et différent en type de la première stimulation ;
un détecteur (59) configuré pour détecter un contact de la peau avec le premier dispositif de fourniture de stimulation (50) et le second dispositif de fourniture de stimulation (60) ;
un dispositif de commande (15) configuré pour commander le premier dispositif de fourniture de stimulation (50) et le second dispositif de fourniture de stimulation (60), sur la base d'un résultat de détection du détecteur (59), et un mécanisme rétractable (90) pour rétracter le premier dispositif de fourniture de stimulation (50) en relation au second dispositif de fourniture de stimulation (60) lorsque la tête de sonde (51) du premier dispositif de fourniture de stimulation (50) est pressée contre la peau, le mécanisme rétractable (90) étant configuré pour stopper la rétractation lorsque le détecteur (59) détecte le contact de la peau avec à la fois le premier dispositif de fourniture de stimulation (50) et le second dispositif de fourniture de stimulation (60).

2. Appareil de soin esthétique (10) selon la revendication 1,
dans lequel le dispositif de commande (15) fait fonctionner le premier dispositif de fourniture de stimulation (50) et le second dispositif de fourniture de stimulation (60) lorsque le détecteur (59) détecte le contact de la peau, et stoppe le fonctionnement du premier dispositif de fourniture de stimulation (50) et du second dispositif de fourniture de stimulation (60) lorsque le détecteur (59) ne détecte pas de contact de la peau.

3. Appareil de soin esthétique (10) selon la revendication 2, dans lequel
lorsque le fonctionnement du premier dispositif de fourniture de stimulation (50) et du second dispositif de fourniture de stimulation (60) se poursuit sur une première durée prédéterminée, le dispositif de commande (15) stoppe le fonctionnement du premier dispositif de fourniture de stimulation et du second dispositif de fourniture de stimulation (60).

4. Appareil de soin esthétique (10) selon l'une quelconque des revendications 1 à 3, comprenant en outre une partie de notification, où le dispositif de commande (15) commande la partie de notification, sur la base d'un résultat de détection du détecteur (59).

5. Appareil de soin esthétique (10) selon l'une quelconque des revendications 1 à 4, comprenant en outre une source d'alimentation qui alimente de l'énergie au premier dispositif de fourniture de stimulation (50) et au second dispositif de fourniture de stimulation (60), où lorsqu'un état dans lequel la source d'alimentation est mise en marche et que le détecteur (59) ne détecte pas le contact de la peau se poursuit sur une seconde durée prédéterminée, le dispositif de commande (15) désactive la source d'alimentation.
